# EUROPEAN PATENT SPECIFICATION

(11) **EP 1 277 840 B1**
(45) Date of publication and mention of the grant of the patent: **11.06.2008**
(21) Application number: 01912513.7
(22) Date of filing: 19.03.2001
(51) Int. Cl.: C12Q 1/06, G01N 27/419

(54) **Drug sensitivity measuring method**
Arzneimittelansprechverhalten-Messverfahren
Méthode de mesure de la sensibilité à un médicament

(30) Priority: 17.03.2000 JP 2000081765
(43) Date of publication of application: 22.01.2003
(73) Proprietor: Jikei University School of Medicine, Tokyo 105-0003 (JP); THE STATE OF JAPAN, as Represented by the DIRECTOR GENERAL of the AGENCY of INDUSTRIAL SCIENCE and TECHNOLOGY, Tokyo 100-9821 (JP); DAIKIN INDUSTRIES, LTD., Osaka-shi, Osaka 530-8323 (JP)
(72) Inventor: MACHIDA, Katsuhiko, Minato-ku Tokyo 105-0003 (JP); HOSHINA, Sadayori, Tokyo 105-0003 (JP); USHIDA, Takashi Nat. Inst. Adv. Interdisciplinary, Tsukuba-shi, Ibaraki 305-8562 (JP); ARAI, Junichiro, Tsukuba-shi Ibaraki 305-0841 (JP); KATAYAMA, Hideo, Tsukuba-shi Ibaraki 305-0841 (JP); OKUMURA, Chiaki, Tsukuba-shi Ibaraki 305-0841 (JP); AMANO, Yoshihisa, Tsukuba-shi Ibaraki 305-0841 (JP)
(74) Representative: Hofer, Dorothea
(86) International application number: PCT/JP2001/002198
(87) International publication number: WO 2001/068905

(56) References cited:
- EP-A- 0 609 458
- EP-A- 0 632 131
- EP-A2- 0 128 527
- JP-A- 10 276 795

## Description

### Technical field

This invention relates to a drug susceptibility measurement method, more particularly, to a method to measure drug susceptibility using an electrode for detection of dissolved oxygen concentration.

### Related art

From past, a method is known for carrying out culturing process by adding bacteria which is a target for measurement to drug solution, as a method for measuring drug susceptibility of the bacteria.

When this method is employed, measurement in drug susceptibility is performed based upon a condition whether or not bacteria proliferation.

When the above method is employed, drug susceptibility should be measured based upon a turbidity of solution, which is increased following the proliferation of bacteria. Therefore, culturing process should be carried out until a timing at which turbidity can be judged so that a disadvantage arises in that an extremely long time period (for example, about 18 hours) is needed.

Especially, in clinical use, when the measurement of drug susceptibility takes a long tome period which is carried out prior to dosage of the drug, the condition of a disease advances during the measurement and a cure rate in decreased. When a drug is given prior to acquisition of the measurement result of the drug susceptibility, resistant bacteria is generated. Powerful drug should be used to get rid of the resistant bacteria so that medical expenses become greater.

The present invention was made in view of the above problems.

It is an object of the present invention to offer a novel drug susceptibility measurement method for rapidly measuring drug susceptibility.

EP-A-0 632 131 and EP-A-0 609 458 disclose drug susceptibility measurement methods on the basis of dissolved oxygen concentration measured by oxygen electrodes. Neither of these methods however measures the current signals for a predetermined time period starting from the peak timing.

### Disclosure of the invention

A drug susceptibility measurement method of claim 1 is a method which has already provided electrodes for detection of dissolved oxygen concentration within a first cell and a second cell, respectively, the first cell containing solvent of measurement drug solution and the second cell containing drug solution. The method then supplies microbe suspension of a predetermined concentration to the first and second cells. The method detects a peak of each signal based upon each signal, each signal being continuously output from each of the electrodes for detection of dissolved oxygen concentration. The method then obtains drug susceptibility based upon the relationship between both signals which are obtained for a predetermined time period starting from the peak timing.

A drug susceptibility measurement method of claim 2 is a method which has already provided electrodes for detection of dissolved oxygen concentration within a first cell and a second cell, respectively the first and second cells containing microbe suspension of a predetermined concentration. The method then supplies solvent of measurement drug solution to the first cell and supplies drug solution to the second cell. The method detects a peak of each signal based upon each signal, each signal being continuously output from each of the electrodes for detection of dissolved oxygen concentration. The method then obtains drug susceptibility based upon the relationship between both signals which are obtained for a predetermined time period starting from the peak timing.

A drug susceptibility measurement method of claim 3 is a method which corrects the both signals so as to determine the peak values of the both signals to be previously determined predetermined values. The method then obtains drug susceptibility based upon the relationship between both corrected signals.

A drug susceptibility measurement method of claim 4 is a method which employs gradient of each signal as the relationship between signals.

When the drug susceptibility measurement method of claim 1 is employed, the method has already provided the electrodes for detection of dissolved oxygen concentration within the first cell and the second cell, respectively, the first cell containing physiological salt solution and the second cell containing drug solution. The method then supplies microbe suspension of a predetermined concentration to the first and second cells. The method detects a peak of each signal based upon each signal, each signal being continuously output from each of the electrodes for detection of dissolved oxygen concentration. The method then obtains drug susceptibility based upon the relationship between both signals which are obtained for a predetermined time period starting from the peak timing. Consequently, drug susceptibility can be measured rapidly based upon the signals for a short time period starting from the peak timing. Therefore, in clinical use, treatment can be carried out by rapidly detecting proper drug (antibiotics etc.). As a result, cure rate is improved and resistant microbe is suppressed in generation.

When the drug susceptibility measurement method of claim 2 is employed, the method has already provided the electrodes for detection of dissolved oxygen concentration within the first cell and the second cell, respectively, the first and second cells containing microbe suspension of a predetermined concentration. The method then supplies solvent of measurement drug solution to the first cell and supplies drug solution to the second cell. The method detects a peak of each signal based upon each signal, each signal being continuously output from each of the electrodes for detection of dissolved oxygen concentration. The method then obtains drug sensitivity based upon the relationship between both signals which are obtained for a predetermined time period starting from the peak timing. Consequently, drug susceptibility can be measured rapidly based upon the signals for a short time period starting from the peak timing. Therefore, in clinical use, treatment can be carried out by rapidly detecting proper drug (antibiotics etc.). As a result, cure rate is improved and resistant microbe is suppressed in generation.

When the drug susceptibility measurement method of claim 3 is employed, the method corrects the both signals so as to determine the peak values of the both signals to be previously determined predetermined values. The method then obtains drug susceptibility based upon the relationship between both corrected signals. Therefore, accuracy in drug susceptibility measurement is improved in addition to the operation and effect of claim 1 or claim 2.

When the drug susceptibility measurement method of claim 4 is employed, the method employs gradient of each signal as the relationship between signals. Therefore, signal processing is simplified improved in addition to the operation and effect of one of claim 1 through claim 3.

### Brief description of the drawings

Figure 1 is a flowchart useful in understanding a drug susceptibility measurement method of an embodiment according to the present invention;
Figure 2 is a diagram representing variation characteristic following passage in time of each of measurement currents when an entire concentration is determined to be 0.25ml;
Figure 3 is a diagram representing variation characteristic following passage in time of each of measurement currents when an entire concentration is determined to be 0.5ml;
Figure 4 is a diagram representing gradients of the measurement currents illustrated in Fig. 2; and
Figure 5 is a diagram representing gradients of the measurement currents illustrated in Fig. 3.

### Best mode for carrying out the invention

Hereinafter, referring to the attached drawings, we explain a drug susceptibility measurement method according to the present invention in detail.

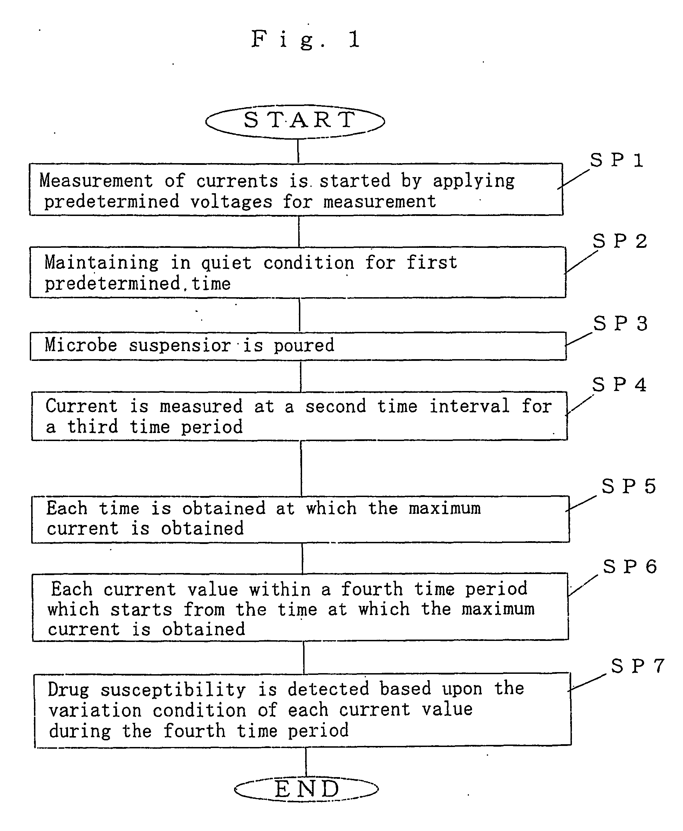

Fig. 1 is a flowchart useful in understanding a drug susceptibility measurement method of an embodiment according to the present invention.

In step SP1, measurement of currents (output signals) is started by applying predetermined voltages for measurement to electrodes for measurement of dissolved oxygen concentration. The electrodes are provided within a first cell, second cell, and third cell, respectively. The first cell is a cell containing only physiological salt solution. The second cell is a cell containing physiological salt solution and drug solution of a first predetermined concentration. The third cell is a cell containing physiological salt solution and drug solution of a second predetermined concentration. In step SP2, those cells are remained in quiet condition for first predetermined time (for t minutes). In step SP3, microbe suspension is poured into each of the cells at a position which is in vicinity to the electrode for measurement of dissolved oxygen concentration. In step SP4, current from each electrode for measurement of dissolved oxygen concentration is measured at a second time interval for a third time period (for example, at every 1 second for 60 seconds). In step SP5, each time is obtained based upon each of the measured currents at which the maximum current is obtained. In step SP6, each current value within a fourth time period which starts from the time at which the maximum current is obtained. In step SP7, drug susceptibility is detected based upon the variation condition of each current value during the fourth time period. Then, the series of operation is finished.

Processing for waiting till a current variation amount for 1 minute becomes equal to or less than a predetermined threshold current (for example, b nA), for example, may be carried out instead the remaining processing in quiet condition for the first predetermined time (for t minutes) which processing is carried out in step SP2. Various factors can be employed as the situation of current variation. It is preferable that current variation rate is employed, from points of view such as simplification in processing.

When the drug susceptibility measurement method corresponding to Fig. 1 is employed, and when microbe suspension is poured into the first, second and third cells and dissolved oxygen concentrations are measured, dissolved oxygen concentration within the first cell is decreased following respiration of microbe. With respect to the first cell, microbe is suppressed its respiration concentration when drug susceptibility exists, while microbe respires without suppressing of respiration concentration when drug susceptibility does not exist, within the second and third cells. Therefore, the second and third cells represent tendency which corresponds to the condition that dissolved oxygen concentration scarcely decreases or the condition that dissolved oxygen concentration decreases similarly to the first cell, responding to the existence and non-existence of drug susceptibility.

Those tendency can be judged within a short time period by using the electrodes for measurement of dissolved oxygen concentration and by using variation condition of each current value during the fourth time period. Therefore, a required time period for measuring drug susceptibility can be greatly decreased. As a result, proper drug is rapidly given so as to improve a cure rate. Further, generation of resistant microbe such as MRSA or the like is suppressed so as to reduce medical expenses.

Next, we explain a specific example.

Ampicillin (hereinafter, referred to as ABPC) is employed as the drug solution, and E. coli {2 species of sensitive strain JM109 and resistant strain 5W (ABPC plasmid introduction)} is employed as the bacteria.

And, physiological salt solution with its concentration of 0.7% is employed as, the concentration of the ABPC solution is adjusted to be 5.3 µg/ml, and the 2 species of E. coli are adjusted to McFarland2.

The physiological salt solution and the drug solution are contained with the ratio represented in table 1, within the first, second and third cells. The bacteria suspension is contained within syringes each provided in correspondence with each cell.

**Table 1**

| | Entire amount | species of bacteria | poured bacteria amount | physiological salt solution | drug solution |
|---|---|---|---|---|---|
| Control | 500 µl | JM109 | 40 µl | 500 µl | 0 µl |
| | 250 µl | JM109 | 20 µl | 250 µl | 0 µl |
| ABPC sensitivity | 500 µl | JM109 | 40 µl | 460 µl | 40 µl |
| | 250 µl | JM109 | 20 µl | 230 µl | 20 µl |
| ABPC resistance | 500 µl | 5W | 40 µl | 460 µl | 40 µl |
| | 250 µl | 5W | 20 µl | 230 µl | 20 µl |

Next, the electrode for measurement of dissolved oxygen concentration is mounted to each cell. The first, second and third cells are maintained quiet condition for 5 minutes so as to make the temperature to be a constant temperature. Syringes containing bacteria solution are set during the above operation. After the time for maintaining quiet condition has passed, the measurement time is determined to be 360 seconds, and the current from each electrode for measurement of dissolved oxygen concentration is measured at every 0.1 seconds. After 5 minutes (300 seconds) has passed, the bacteria suspension is poured into each cell from each syringe rapidly at a constant speed. After the measurement time of 360 seconds has passed, the measured currents are stored and the measured currents are analyzed. This analysis was carried out in the following manner.

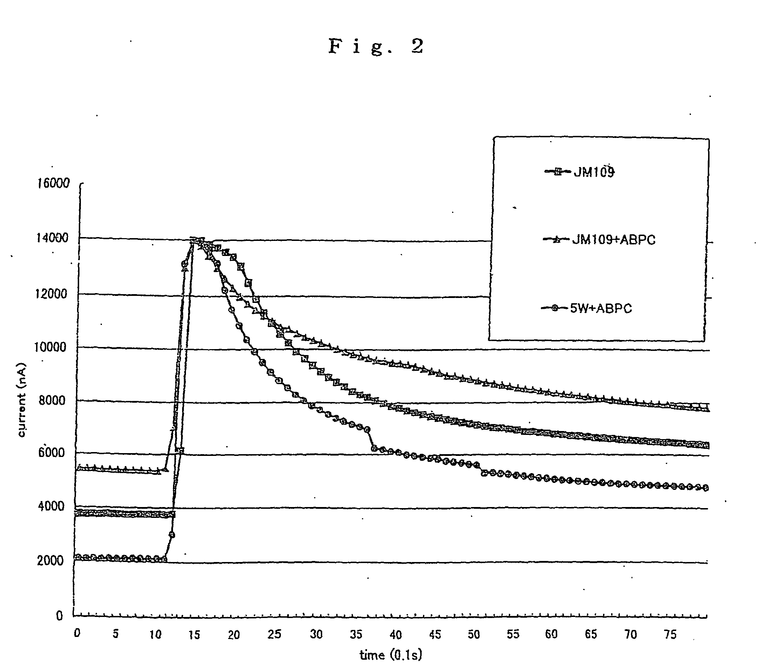

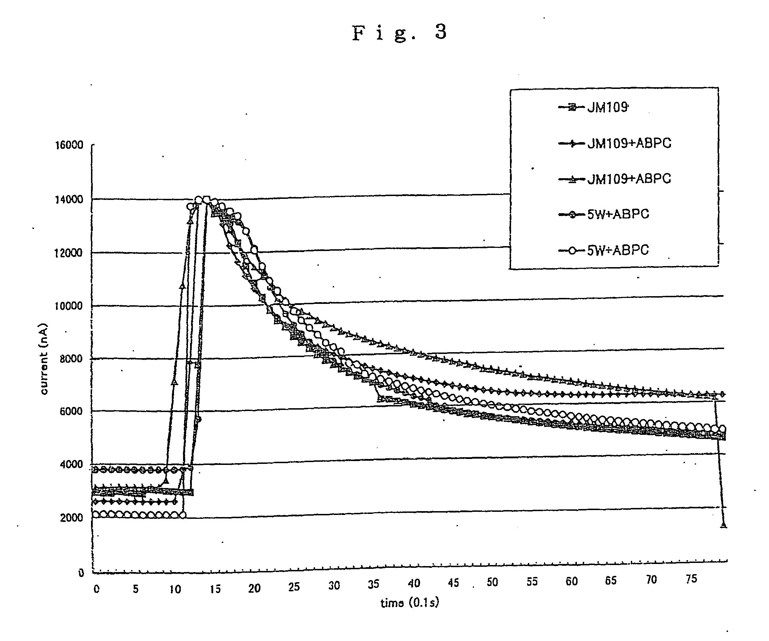

The entirety of a series of each of the measured currents is displaced in parallel so as to match the peak point of each series of each of the measured current to the position corresponding to 1.7 seconds. And, figures representing variation characteristic following passage in time are obtained (refer to Fig. 2 representing variation characteristic following passage in time of each of measurement currents when an entire amount is determined to be 0.25ml, and Fig. 3 representing variation characteristic following passage in time of each of measurement currents when an entire amount is determined to be 0.5ml).

When theoretical consideration is carried out, at first, bacteria is determined to be a control, and the sensitive bacteria and the resistant bacteria are compared. When bacteria is resistant bacteria, measured current variation characteristic following passage in time which is nearly equal to the control should be obtained because the bacteria scarcely affected by drug so as to scarcely varied oxygen consumption concentration. On the contrary, when bacteria is sensitive bacteria, oxygen consumption concentration is decreased, measured current variation characteristic following passage in time should be more gentle with respect to the control, because the bacteria is interfered its respiration by pouring the drug.

In the actual measurement results illustrated in Figs. 2 and 3, When sensitive "JM109 + ABPC" and resistant "5W + ABPC" are compared by determining the control "JM109" as the reference, the actual measurement results illustrated in Figs. 2 and 3a represent that a curve corresponding to the sensitive strain is more gentle than curves each corresponding to the control and the resistant strain, and a curve corresponding to the resistant strain is equal to or more steep than the curve corresponding to the control.

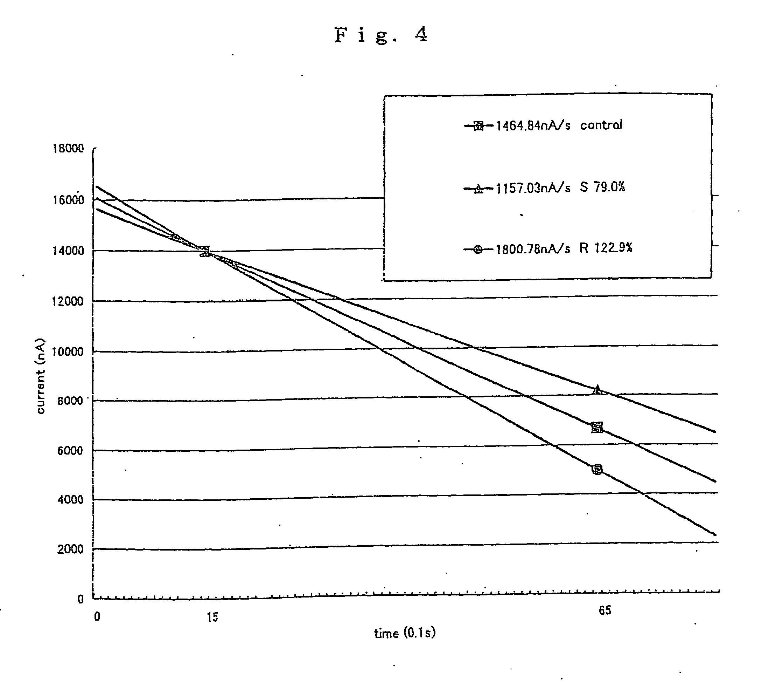

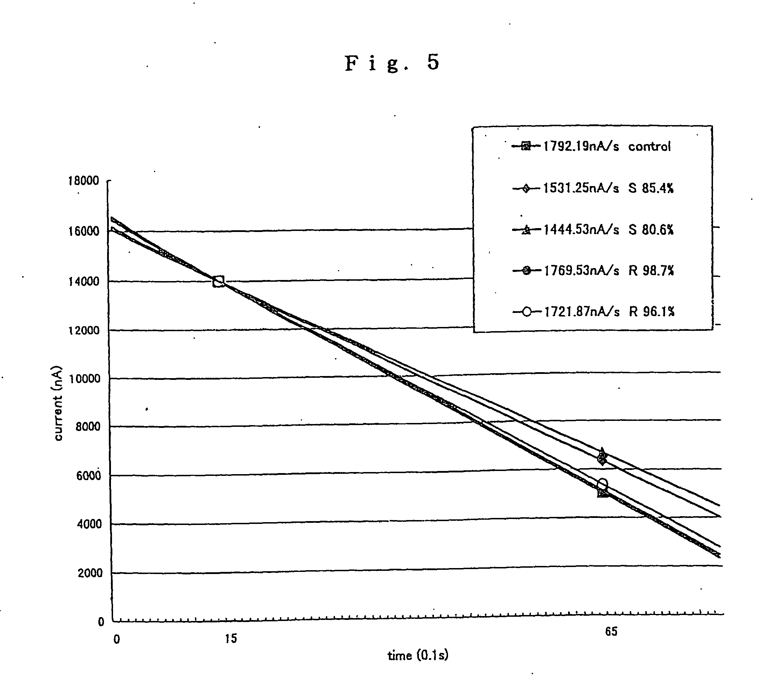

To digitize the measurement results illustrated in Figs. 2 and 3, a difference between the current value at the timing 10 seconds having passed from the peak timing and the peak current value is divided by the passage of time so as to obtain the inclination for 10 seconds. As a result, graphs illustrated in Figs. 4 and 5 are obtained.

When each inclination is compared by supposing the inclination corresponding to the control to be 100%, the inclination corresponding to the sensitive strain is 79.0 - 85.4%, while the inclination corresponding to the resistant strain is 96.1-122.9%.

Therefore, it is sufficient that the threshold value is determined to be 90% when the inclination corresponding to the control is determined to be 100%. Judgement can be made that bacteria is sensitive strain when the inclination is less than 90%, and that bacteria is resistant strain when the inclination is equal to or greater than 90%.

The sensitivity judgement may be carried out based upon an inclination for 5 seconds after the peak timing. The sensitivity judgement may also be carried out based upon a tendency in measurement current other than the inclination.

The invention of claim 1 has characteristic operation and effect such that drug susceptibility can be measured rapidly based upon the signals for a short time period starting from the peak timing, that treatment can be carried out by rapidly detecting proper drug (antibiotics etc.) in clinical use, and that cure rate is improved and resistant microbe is suppressed in generation.

The invention of claim 2 has characteristic operation and effect such that drug susceptibility can be measured rapidly based upon the signals for a short time period starting from the peak timing, that treatment can be carried out by rapidly detecting proper drug (antibiotics etc.) in clinical use, and that cure rate is improved and resistant microbe is suppressed in generation.

The invention of claim 3 has characteristic operation and effect such that accuracy in drug susceptibility measurement is improved in addition to the operation and effect of claim 1 or claim 2.

The invention of claim 4 has characteristic operation and effect such that signal processing is simplified improved in addition to the operation and effect of one of claim 1 through claim 3.

## Claims

1. A drug susceptibility measurement method which has already provided electrodes for detection of dissolved oxygen concentration within a first cell and a second cell, respectively, the first cell containing solvent of drug solution and the second cell containing drug solution, the method comprising the steps of:
supplying microbe suspension of a predetermined concentration into the first and second cells; detecting a peak of each current signal based upon each current signal, each current signal being continuously output from each of the electrodes for detection of dissolved oxygen concentration; and
obtaining drug susceptibility based upon the relationship between both current signals which are obtained for a predetermined time period starting from the peak timing.

2. A drug susceptibility measurement method which has already provided electrodes for detection of dissolved oxygen concentration within a first cell and a second cell, respectively, the first and second cells containing microbe suspension of a predetermined concentration, the method comprising the steps of:
supplying solvent of drug solution to the first cell and supplies drug solution to the second cell; detecting a peak of each current signal based upon each current signal, each current signal being continuously output from each of the electrodes for detection of dissolved oxygen concentration; and
obtaining drug susceptibility based upon the relationship between both current signals which are obtained for a predetermined time period starting from the peak timing.

3. A drug susceptibility measurement method as set forth in claim 1 or claim 2, wherein the both current signals are displaced in parallel so as to determine the peak values of the both current signals to be previously determined predetermined values, and drug susceptibility is obtained based upon the relationship between both corrected current signals.

4. A drug susceptibility measurement method as set forth in one of claim 1 through claim 3, wherein a relationship between gradients of each current signal is employed as the relationship between current signals.

## Patentansprüche

1. Arzneimittelansprechverhalten-Meßverfahren, das Elektroden zur Detektion einer Konzentration an gelöstem Sauerstoff innerhalb jeweils einer ersten Zelle und einer zweiten Zelle bereits bereitgestellt aufweist, wobei die erste Zelle Lösungsmittel einer Arzneimittellösung enthält und die zweite Zelle Arzneimittellösung enthält, wobei das Verfahren die Schritte umfasst:
Einbringen einer Mikrobensuspension einer festgelegten Konzentration in die ersten und zweiten Zellen;
Detektieren eines auf jedem Stromsignal basierenden Peaks von jedem Stromsignal, wobei jedes Stromsignal kontinuierlich von jeder der Elektroden zur Detektion einer Konzentration an gelöstem Sauerstoff ausgegeben wird; und
Erhalten eines Arzneimittelansprechverhaltens, das auf dem Verhältnis zwischen beiden Stromsignalen basiert, die für eine vom Peakzeitpunkt beginnende festgelegte Zeitdauer erhalten werden.

2. Arzneimittelansprechverhalten-Meßverfahren, das Elektroden zur Detektion einer Konzentration an gelöstem Sauerstoff innerhalb jeweils einer ersten Zelle und einer zweiten Zelle bereits bereitgestellt aufweist, wobei die erste Zelle Lösungsmittel einer Arzneimittellösung enthält und die zweite Zelle Arzneimittellösung enthält, wobei das Verfahren die Schritte umfasst:
Einbringen von Lösungsmittel einer Arzneimittellösung in die erste Zelle und Einbringen von Arzneimittellösung in die zweite Zelle;
Detektieren eines auf jedem Stromsignal basierenden Peaks von jedem Stromsignal, wobei jedes Stromsignal kontinuierlich von jeder der Elektroden zur Detektion einer Konzentration an gelöstem Sauerstoff ausgegeben wird; und
Erhalten eines Arzneimittelansprechverhaltens, das auf dem Verhältnis zwischen beiden Stromsignalen basiert, die für eine vom Peakzeitpunkt beginnende festgelegte Zeitdauer erhalten werden.

3. Arzneimittelansprechverhalten-Meßverfahren wie in Anspruch 1 oder Anspruch 2 dargelegt, wobei die beiden Stromsignale parallel verschoben werden, um die Peakwerte der beiden Stromsignale so festzulegen, dass sie vorher bestimmte festgelegte Werte sind, und wobei das Arzneimittelansprechverhalten basierend auf dem Verhältnis zwischen beiden korrigierten Stromsignalen erhalten wird.

4. Arzneimittelansprechverhalten-Meßverfahren wie in einem von Anspruch 1 bis Anspruch 3 dargelegt, wobei ein Verhältnis zwischen Steigungen jedes Stromsignals als das Verhältnis zwischen Stromsignalen genutzt wird.

## Revendications

1. Procédé de mesure de la sensibilité à un médicament qui est déjà pourvu d'électrodes pour une détection de la concentration d'oxygène dissous à l'intérieur d'une première cellule et d'une deuxième cellule, respectivement, la première cellule contenant un solvant d'une solution médicamenteuse et la deuxième cellule contenant une solution médicamenteuse, le procédé comprenant les étapes consistant à :
injecter une suspension microbienne à une concentration prédéterminée dans les première et deuxième cellules ;
détecter un pic pour chaque signal de courant en se basant sur chaque signal de courant, chaque signal de courant étant généré en continu à partir de chacune des électrodes servant à une détection de la concentration d'oxygène dissous ; et
obtenir une sensibilité à un médicament en se basant sur la relation entre les deux signaux de courant qui sont obtenus au cours d'une période de temps prédéterminée qui débute au moment du pic.

2. Procédé de mesure de la sensibilité à un médicament qui est déjà pourvu d'électrodes pour une détection de la concentration d'oxygène dissous à l'intérieur d'une première cellule et d'une deuxième cellule, respectivement, les première et deuxième cellules contenant une suspension microbienne à une concentration prédéterminée, le procédé comprenant les étapes consistant à :
injecter un solvant d'une solution médicamenteuse dans la première cellule, et injecter une solution médicamenteuse dans la deuxième cellule ; détecter un pic pour chaque signal de courant en se basant sur chaque signal de courant, chaque signal de courant étant généré en continu à partir de chacune des électrodes servant à une détection de la concentration d'oxygène dissous ; et
obtenir une sensibilité à un médicament en se basant sur la relation entre les deux signaux de courant qui sont obtenus au cours d'une période de temps prédéterminée qui débute au moment du pic.

3. Procédé de mesure de la sensibilité à un médicament selon la revendication 1 ou 2, dans lequel les deux signaux de courant se déplacent en parallèle de façon à déterminer les valeurs de pic des deux signaux de courant de façon à ce qu'elles soient des valeurs prédéterminées préalablement déterminées, et une sensibilité à un médicament est obtenue en se basant sur la relation entre les deux signaux de courant corrigés.

4. Procédé de mesure de la sensibilité à un médicament selon l'une des revendications 1 à 3, dans lequel une relation entre des gradients de chaque signal de courant est employée en tant que la relation entre des signaux de courant.
